# EUROPEAN PATENT SPECIFICATION

(11) **EP 2 206 984 B1**
(45) Date of publication and mention of the grant of the patent: **21.03.2012**
(21) Application number: 10150424.9
(22) Date of filing: 11.01.2010
(51) Int. Cl.: F24F 11/00

(54) **Air conditioner and method for controlling the same**
Klimaanlage und Steuerverfahren dafür
Climatiseur et procédé de commande correspondant

(30) Priority: 12.01.2009 KR 20090002158
(43) Date of publication of application: 14.07.2010
(73) Proprietor: LG Electronics Inc., Seoul 150-721 (KR)
(72) Inventor: Lee, Ju Youn, 153-802, Seoul (KR); Chung, Baik Young, 153-802, Seoul (KR); Jang, Jae Dong, 153-802, Seoul (KR)
(74) Representative: Vossius & Partner

(56) References cited:
- EP-A1- 1 998 118
- JP-A- 2006 317 074
- JP-A- 2008 304 181

## Description

The present disclosure relates to an air conditioner and, more particularly, to an air conditioner that can control indoor air for user's comfortable sleep and a method for controlling the air conditioner.

An air conditioner is an appliance that cools or heats an indoor space. However, the air conditioners of related arts have a limitation in that they cannot fulfill a variety of requirements of sleepers.

JP 2006-317074 A discloses controlling an indoor temperature a-ccording to a rapid eye movement cycle. The temperature in a period between the rapid eye movement operations is not kept constant but is increased or decreased.

The present invention defined in the claims provides an air conditioner and a method for controlling the air conditioner that can allow a sleeper to more efficiently get a sleep.

The details of one or more embodiments are set forth in the accompanying drawings and the description below. Other features will be apparent from the description and drawings, and from the claims.

### BRIEF DESCRIPTION OF THE DRAWINGS

Fig. 1 is a perspective view of an air conditioner according to an embodiment.

Fig. 2 is a block diagram of an air conditioner according to an embodiment.

Figs. 3 and 4 are flowcharts illustrating a method for controlling an air conditioner according to an embodiment.

Fig. 5 is a graph illustrating a temperature variation according to an embodiment.

Fig. 6 is a graph illustrating a brainwave of a sleep state of a user when indoor air is conditioned by a related art.

Fig. 7 is a graph illustrating a brainwave of a sleep state of a user when indoor air is conditioned by an embodiment.

### DETAILED DESCRIPTION OF THE EMBODIMENTS

Reference will now be made in detail to the embodiments of the present disclosure, examples of which are illustrated in the accompanying drawings.

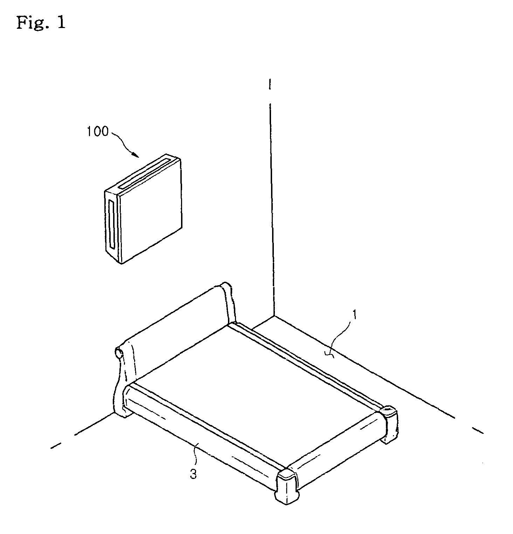

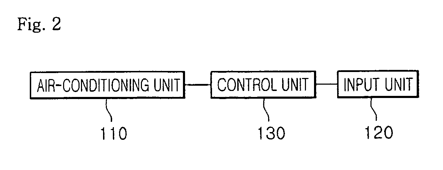

Fig. 1 is a perspective view of an air conditioner according to an embodiment and Fig. 2 is a block diagram of an air conditioner according to an embodiment.

Referring first to Fig. 1, an air conditioner 100 of an embodiment may be installed at a side of an indoor space 1. In Fig. 1, the air conditioner 100 is installed on a wall surface above a head portion of a bed 3.

Referring to Fig. 2, the air conditioner 100 includes an air-conditioning unit 110, an input unit 120, and a control unit 130. The air-conditioning unit 110 includes a variety of components such as a compressor, an indoor unit, and the like for air-conditioning a bedroom. Since a structure of the air-conditioning unit is well known in the art, detailed description of the air-conditioning unit will be omitted herein.

The input unit 120 signals such as, for example, a signal for setting a cooling temperature and a signal for controlling an air volume. In addition, the input unit 120 receives signal for setting a sleep mode. For example, the input unit 120 receives signals for selecting the sleep mode, and setting a sleep time in an hour or minute unit.

The control unit 130 controls the air-conditioning unit 110 in accordance with the signal input to the input unit 120. That is, the control unit 130 controls the air-conditioning unit 110 in accordance with the air-conditioning temperature and time input to the input unit 120.

When the signal for selecting the sleep mode and the signal for setting the sleep time are input to the input unit 120, the control unit 130 controls such that the air-conditioning unit performs a sleep entry operation, a sleep operation, a first rapid eye movement (REM) sleep operation, a sleep operation, and a second REM sleep operation. The REM sleep means a sleep accompanying a rapid eye movement. Generally, the sleeps are classified into REM sleeps and non-REM sleeps. The REM sleep is kept for a predetermined time at intervals of a predetermined time. Generally, the REM sleep is kept for 20-40 minutes at intervals of 90-110 minutes. However, the intervals and time of the REM sleep may be different depending on sleepers. It is well known that sufficient sleep effect cannot be attained when the sleeper can not get a deep sleep for the REM sleep.

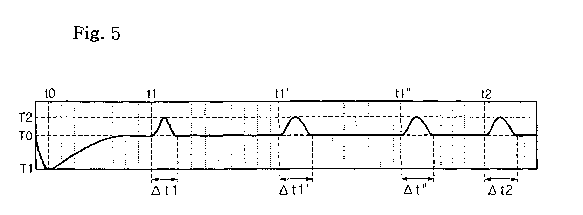

In more detail, when the sleep time input to the input unit 120 initiates, the control unit 130 controls such that the air-conditioning unit 110 performs the sleep entry operation until it reaches a preset sleep entry time t0. The control unit 130 then controls such that the air-conditioning unit 110 performs the sleep operation until the first REM sleep time t1. When it reaches the first REM sleep time t1, the control unit 130 controls such that the air-conditioning unit 110 performs the first REM sleep operation for a preset first REM sleep operation time dt1. When the first REM sleep operation time dt1 has elapsed, the control unit 130 controls such that the air-conditioning unit 10 repeatedly performs the sleep operation and first REM sleep operation by a plurality of times (2 times in Fig. 5). Meanwhile, when the air-6 conditioning unit 110 completes finally the first REM sleep operation, the control unit 130 controls such that the air-conditioning unit 110 performs the sleep operation until it reaches a second REM sleep time t2. When it reaches the second REM sleep time t2, the control unit 130 controls such that the air-conditioning unit 110 performs the second REM sleep operation for a preset second REM sleep operation time dt2. When the second REM sleep operation time has elapsed, the sleep mode is performed until the sleep time has elapsed.

Meanwhile, the control unit 130 controls such that the air conditioning unit 110 air-conditions the indoor space 1 at predetermined sleep entry temperature T1, sleep temperature T0, and REM sleep temperature T2 in the respective sleep entry operation, sleep operation, and first and second REM sleep operations. Here, the sleep temperature T0 is set to be same as a temperature of the sleep mode input to the input unit 120. Further, the sleep entry temperature T1 is set to be less than the sleep temperature T0 and the REM sleep temperature T2 is set to be higher than the sleep temperature T0. That is, since the sleep initiates in the sleep entry operation, the sleeper can more quickly fall asleep by setting the sleep entry temperature T1 less than the sleep temperature T0. Since the autonomic nerve of the sleeper cannot be efficiency controlled during the REM sleep, the body temperature of the sleeper can be kept by setting the REM sleep temperature T2 higher than the sleep temperature T0. In this embodiment, the sleep entry temperature T1 is set to be less than the sleep temperature T0 by 1-3°C, preferably 2°C. The REM sleep temperature T2 is set to be higher than the sleep temperature T0 by 0.5-1.5°C, preferably 1.0°C.

The sleep entry time t0 is a time which the sleeper takes to get a sleep after the sleep time initiates. In this embodiment, the sleep entry time t0 is set as a time when 5-15 minutes, preferably 10 minutes, after the sleep time initiates has elapsed.

The first and second REM sleep times t1, t1' t1", and t2 and the first and second REM sleep operation times dt1 and dt2 can be set according to the above-described REM sleep features. That is, the first and second REM sleep times t1, t1' t1", and t2 and the first and second REM sleep operation times dt1 and dt2 may be set corresponding to the REM sleep intervals and times of the respective sleepers. In this embodiment, the first REM sleep times t1, t1', and t1" are set as times at intervals of 9-110 minutes, preferably 100 minutes. The second REM sleep time t2 is set as a time after 60-8 minutes, preferably 70 minutes, have elapsed after the final first REM sleep time t1" among the first REM sleep times t1, t1' and t1". In addition, the first and second REM sleep operation times dt1 and dt2 are set as 20-40 minutes, preferably 30 minutes.

The following will describe a method for controlling an air conditioner according to an embodiment in more detail.

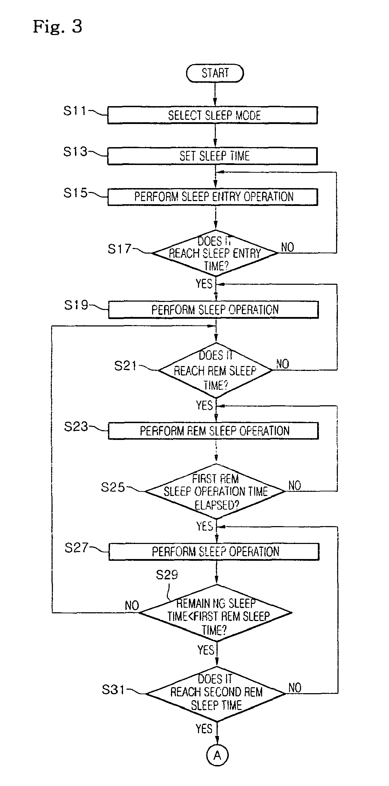

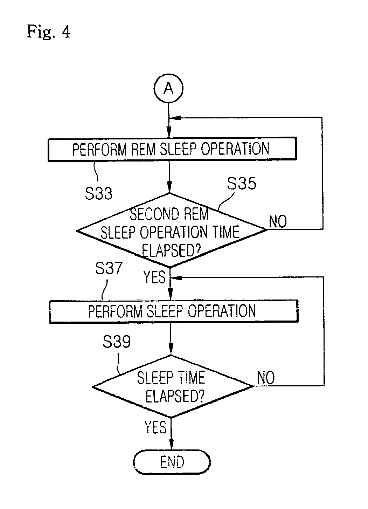

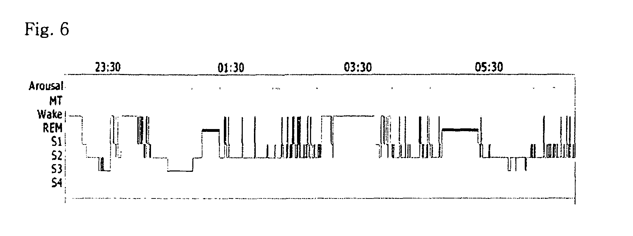

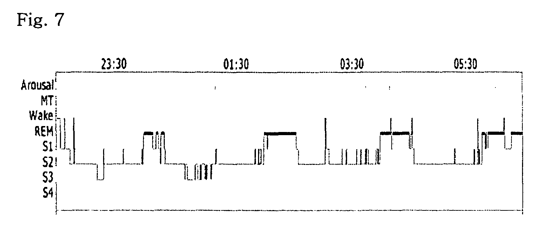

Figs. 3 and 4 are flowcharts illustrating a method for controlling an air conditioner according to an embodiment, Fig. 5 is a graph illustrating a temperature variation according to an embodiment, Fig. 6 is a graph illustrating a brainwave of a sleep state of a user when indoor air is conditioned by a related art, and Fig. 7 is a graph illustrating a brainwave of a sleep state of a user when indoor air is conditioned by an embodiment.

Referring first to Figs. 3 and 4, the input unit 120 receives a signal for selecting a sleep mode (S11). Next, the input unit 120 receives signals for setting the sleep temperature T0 and sleep time (S13). In this embodiment, the sleep temperature T0 is 26°C and the sleep time is 390 minutes. However, the present invention is not limited to this.

Meanwhile, when the input unit 120 receives the signals for selecting the sleep mode and the sleep time, the control unit 130 controls such that the air-conditioning unit 110 performs the sleep entry operation (S15). Therefore, the indoor space 1 is air-conditioned at the sleep entry temperature T1 of 24°C until it reaches the sleep entry time t0 after the sleep time initiates, i.e., for 10 minutes.

Further, the control unit 130 determines if it reaches the sleep entry time t0 (S17). When it is determined that is reaches the sleep entry time t0, the control unit 130 controls such that the air-conditioning unit 110 performs the sleep operation (S19). Therefore, the indoor space 1 is air-conditioned at the sleep temperature T0 of 26°C.

Next, the control unit 130 determines if it reaches the first REM sleep time t1 (S21). When it is determined that it reaches the first REM sleep time t1, the control unit 130 controls such that the air-conditioning unit 110 performs the first REM sleep operation (S23). Therefore, the indoor space 1 is air-conditioned for the first REM sleep operation time dt1 at the temperature T2 of 27°C.

Further, the control unit 130 determines if the first REM sleep operation time dt1 has elapsed (S25). When it is determined that the first REM sleep operation time dt1 has elapsed, the control unit 130 controls such that the air-conditioning unit 110 performs the sleep operation (S27).

Meanwhile, the control unit 130 determines if a remaining sleep time at present is less than the first REM sleep times t1' and t1" (S29). When it is determined that the remaining sleep time at present is less than the first REM sleep times t1' and t1", the control unit 130 determines if it reaches the second REM sleep time t2 (S31). When it is determined that it reaches the second REM sleep time t2 (S31), the control unit 130 controls such that the air-conditioning unit performs the second REM sleep operation (S33). Accordingly, the indoor space 1 is air-conditioned at the REM sleep temperature T2 of 27°C for the second REM sleep operation time dt2.

The control unit 130 determines if the second REM sleep operation time dt2 has elapsed (S35). When it is determined that the second REM sleep operation time dt2 has elapsed, the control unit 130 controls such that the air-conditioning unit 110 performs the sleep operation (S37).

Finally, the control unit 130 determines if the sleep time has elapsed (S39). When it is determined that the sleep time has elapsed, the sleep mode is finished.

Meanwhile, when it is determined in Step 29 that the remaining sleep time is equal to or greater than the first REM sleep times t1' and t1" Steps 21 to 27, i.e., the first REM sleep operation and sleep operation are performed. The repeated performance of the first REM sleep operation and the sleep operation is continued it is determined that the remaining sleep time is less than the first REM sleep times t1' and t1".

When the sleep mode is performed as described above, the temperatures of the indoor space 1 are as shown in Fig. 5. That is, the temperature of the indoor space in the sleep entry operation is reduced. When the sleep entry operation is finished, the temperature of the indoor space 1 increases and maintains a predetermined level. Further, in the first and second REM sleep operations, the temperature of the indoor space 1 increases again.

Fig. 6 shows a graph illustrating a brainwave of a sleep state of a user when indoor air is conditioned by a related art, and Fig. 7 shows a graph illustrating a brainwave of a sleep state of a user when indoor air is conditioned by the embodiment. The brainwave of the user in the graph of Fig. 7 shows that the wakeup state of the user is significantly reduced as compared with the graph of Fig. 6.

As described above, according to the air conditioner and the method for controlling the air condition of the embodiments, the sleeper can more efficiently get the sleep especially in the REM sleep.

Although embodiments have been described with reference to a number of illustrative embodiments thereof, it should be understood that numerous other modifications and embodiments can be devised by those skilled in the art that will fall within the scope of
the appended claims. In addition to variations and modifications in the component parts and/or arrangements, alternative uses will also be apparent to those skilled in the art.

## Claims

1. An air conditioner (100) comprising an air-conditioning unit (110) for air-conditioning of an indoor space, an input unit (120) receiving signals for operating the air-conditioning unit (110), and a control unit (130) for controlling the air-conditioning unit (110) in accordance with the signals input to the input unit (120),
wherein the input unit (120) receives a signal for selecting a sleep mode,
the control unit (130) controls such that, when the input unit (120) receives the signal for selecting the sleep mode, the air-conditioning unit (110) performs a sleep entry operation, a sleep operation and a rapid eye movement sleep operation, the sleep operation and the rapid eye movement sleep operation being alternately repeatedly performed, and
the sleep operation is performed for a preset time at a temperature (T0) that is set in accordance with the sleep mode, the sleep entry operation is performed for a preset time at a temperature less than the temperature (T0) of the sleep operation, the rapid eye movement sleep operation is performed for a preset time at a temperature higher than the temperature (T0) of the sleep operation, the preset time of the rapid eye movement sleep operation is shorter than the preset time of the sleep operation, and the temperature (T0) of the sleep operation is kept constant between the rapid eye movement sleep operations.

2. The air conditioner according to claim 1, **characterized in that** the rapid eye movement sleep operation is performed for a time corresponding to a rapid eye movement cycle in a process for performing the sleep mode.

3. The air conditioner according to any one of claims 1 and 2, **characterized in that** the rapid eye movement sleep operation is performed may times at preset time intervals.

4. The air conditioner according to any one of claims 1 to 2, **characterized in that** the rapid eye movement sleep operation is performed many times at intervals of 60-110 minutes in a process for performing the sleep operation.

5. The air conditioner according to any one of claims 1 to 4, **characterized in that** the rapid eye movement sleep operation comprises:
a first rapid eye movement sleep operation that is performed at least one time at intervals of 90-100 minutes during a process for performing the sleep mode; and
a second rapid eye movement sleep operation that is performed at least onetime at intervals of 60-80 minutes after the first rapid eye movement sleep operation is finished.

6. The air conditioner according to any one of claims 1 to 5, **characterized in that**, in the rapid eye movement sleep operation, the air-conditioning unit (110) air-conditions the indoor space at a temperature higher than that in the sleep operation by 1 °C.

7. The air conditioner according to any one of claims 1 to 6, **characterized in that** the rapid eye movement sleep operation is performed for 20-40 minutes.

8. The air conditioner according to any one of claims 1 to 7, **characterized in that**, in the sleep entry operation, the air-conditioning unit (110) air-conditions the indoor space at a temperature lower than that in the sleep operation by 2°C.

9. The air conditioner according to any one of claims 1 to 7, **characterized in that** the sleep entry operation is performed for 5-15 minutes.

10. A method for controlling an air conditioner (100) comprising an air-conditioning unit (110) for air-conditioning of an indoor space, an input unit (120) receiving signals for setting air-conditioning temperature and time for the indoor space, and a control unit (130) for controlling operation of the air-conditioning unit (110), the method comprising :
allowing the air-conditioning unit (110) to air-condition the indoor space at a temperature less than the air-conditioning temperature (T0) in a sleep entry step;
allowing the air-conditioning unit (110) to air-condition the indoor space at the air-conditioning temperature (T0) in a sleep operation step; and
allowing the air-conditioning unit (110) to air-condition the indoor space at a rapid eye movement sleep temperature higher than the air-conditioning temperature (T0) in a rapid eye movement sleep operation step,
wherein the sleep operation step and the rapid eye movement sleep operation step are alternately repeatedly performed until the operation time has elapsed,
the sleep operation step is performed for a preset time at a temperature (T0), the rapid eye movement sleep operation step is performed for a preset time, the preset time of the rapid eye movement sleep operation step is shorter than the preset time of the sleep operation step, and the temperature (T0) is kept constant during the sleep operation step between the rapid eye movement sleep operation steps.

11. The method according to claim 10, **characterized in that** the sleep entry temperature is less than the air-conditioning temperature by 2°C; and
the rapid eye movement sleep temperature is higher than the air-conditioning temperature by 1°C.

12. The method according to any one of claims 10 and 11, **characterized in that** the sleep entry operation step is performed for 5-15 minutes.

13. The method according to any one of claims 10 to 12, **characterized in that** the rapid eye movement sleep operation step comprises:
a first rapid eye movement sleep operation step that is performed at least one time at intervals of 90-100 minutes during a process for performing the sleep mode; and
a second rapid eye movement sleep operation step that is performed at least one time at intervals of 60-80 minutes after the first rapid eye movement sleep operation is finished.

14. A method according to any one of claims 10 to 13, **characterized in that** the rapid eye movement sleep operation step is performed for 20-40 minutes.

## Patentansprüche

1. Klimaanlage (100) mit einer Klimatisierungseinheit (110) zum Klimatisieren eines Innenraums, einer Eingabeeinheit (120), die Signale für den Betrieb der Klimatisierungseinheit (110) empfängt, und einer Steuereinheit (130) zur Steuerung der Klimatisierungseinheit (110) gemäß den in die Eingabeeinheit (120) eingegebenen Signalen,
wobei die Eingabeeinheit (120) ein Signal zum Wählen eines Schlafmodus empfängt,
die Steuereinheit (130) eine solche Steuerung vornimmt, dass, wenn die Eingabeeinheit (120) das Signal zum Wählen des Schlafmodus empfängt, die Klimatisierungseinheit (110) einen Schlafeintrittsbetrieb, einen Schlafbetrieb und einen REM-Schlafbetrieb (REM: rapid eye movement, rasche Augenbewegung) durchführt, wobei der Schlafbetrieb und der REM-Schlafbetrieb wiederholt abwechselnd durchgeführt werden, und
der Schlafbetrieb für eine vorgegebene Zeit bei einer Temperatur (T0), die gemäß dem Schlafmodus festgesetzt ist, durchgeführt wird, der Schlafeintrittsbetrieb für eine vorgegebene Zeit bei einer Temperatur, die niedriger ist als die Temperatur (T0) des Schlafbetriebs, durchgeführt wird, der REM-Schlafbetrieb für eine vorgegebene Zeit bei einer Temperatur, die höher ist als die Temperatur (T0) des Schlafbetriebs, durchgeführt wird, die vorgegebene Zeit des REM-Schlafbetriebs kürzer ist als die vorgegebene Zeit des Schlafbetriebs und zwischen den REM-Schlafbetrieben die Temperatur (T0) des Schlafbetriebs konstant gehalten wird.

2. Klimaanlage nach Anspruch 1, **dadurch gekennzeichnet, dass** in einem Prozess zur Durchführung des Schlafmodus der REM-Schlafbetrieb für eine Zeit durchgeführt wird, die einem REM-Zyklus entspricht.

3. Klimaanlage nach einem der Ansprüche 1 und 2, **dadurch gekennzeichnet, dass** der REM-Schlafbetrieb mehrere Male in vorgegebenen Zeitabständen durchgeführt wird.

4. Klimaanlage nach einem der Ansprüche 1 bis 2, **dadurch gekennzeichnet, dass** in einem Prozess zur Durchführung des Schlafbetriebs der REM-Schlafbetrieb mehrere Male in Abständen von 60-110 Minuten durchgeführt wird.

5. Klimaanlage nach einem der Ansprüche 1 bis 4, **dadurch gekennzeichnet, dass** der REM-Schlafbetrieb aufweist:
einen ersten REM-Schlafbetrieb, der während eines Prozesses zur Durchführung des Schlafmodus mindestens ein Mal in Abständen von 90-100 Minuten durchgeführt wird; und
einen zweiten REM-Schlafbetrieb, der nach Beendigung des ersten REM-Schlafbetriebs mindestens ein Mal in Abständen von 60-80 Minuten durchgeführt wird.

6. Klimaanlage nach einem der Ansprüche 1 bis 5, **dadurch gekennzeichnet, dass** in dem REM-Schlafbetrieb die Klimatisierungseinheit (110) den Innenraum auf eine Temperatur bringt, die um 1°C höher ist als in dem Schlafbetrieb.

7. Klimaanlage nach einem der Ansprüche 1 bis 6, **dadurch gekennzeichnet, dass** der REM-Schlafbetrieb für 20-40 Minuten durchgeführt wird.

8. Klimaanlage nach einem der Ansprüche 1 bis 7, **dadurch gekennzeichnet, dass** in dem Schlafeintrittsbetrieb die Klimatisierungseinheit (110) den Innenraum auf eine Temperatur bringt, die um 2°C niedriger ist als in dem Schlafbetrieb.

9. Klimaanlage nach einem der Ansprüche 1 bis 7, **dadurch gekennzeichnet, dass** der Schlafeintrittsbetrieb für 5-15 Minuten durchgeführt wird.

10. Verfahren zur Steuerung einer Klimaanlage (100), die eine Klimatisierungseinheit (110) zum Klimatisieren eines Innenraums, eine Eingabeeinheit (120), die Signale zum Einstellen von Klimatisierungstemperatur und -Zeit für den Innenraum empfängt, und eine Steuereinheit (130) zur Steuerung des Betriebs der Klimatisierungseinheit (110) aufweist, wobei das Verfahren die folgenden Schritte aufweist:
einen Schlafeintrittsschritt, in welchem die Klimatisierungseinheit (110) den Innenraum auf eine Temperatur bringt, die niedriger ist als die Klimatisierungstemperatur (T0);
einen Schlafbetriebsschritt, in welchem die Klimatisierungseinheit (110) den Innenraum auf die Klimatisierungstemperatur (T0) bringt; und
einen REM-Schlafbetriebsschritt, in welchem die Klimatisierungseinheit (110) den Innenraum auf eine REM-Schlaftemperatur bringt, die höher ist als die Klimatisierungstemperatur (T0),
wobei der Schlafbetriebsschritt und der REM-Schlafbetriebsschritt wiederholt abwechselnd durchgeführt werden, bis die Betriebszeit verstrichen ist,
der Schlafbetriebsschritt für eine vorgegebene Zeit bei einer Temperatur (T0) durchgeführt wird, der REM-Schlafbetriebsschritt für eine vorgegebene Zeit durchgeführt wird, die vorgegebene Zeit des REM-Schlafbetriebsschritts kürzer ist als die vorgegebene Zeit des Schlafbetriebsschritts, und während des Schlafbetriebsschritts die Temperatur (T0) zwischen den REM-Schlafbetriebsschritten konstant gehalten wird.

11. Verfahren nach Anspruch 10, **dadurch gekennzeichnet, dass** die Schlafeintrittstemperatur um 2°C niedriger ist als die Klimatisierungstemperatur; und
die REM-Schlaftemperatur um 1°C höher ist als die Klimatisierungstemperatur.

12. Verfahren nach einem der Ansprüche 10 und 11, **dadurch gekennzeichnet, dass** der Schlafeintrittsschritt für 5-15 Minuten durchgeführt wird.

13. Verfahren nach einem der Ansprüche 10 bis 12, **dadurch gekennzeichnet, dass** der REM-Schlafbetriebsschritt aufweist:
einen ersten REM-Schlafbetriebsschritt, der während eines Prozesses zur Durchführung des Schlafmodus mindestens ein Mal in Abständen von 90-100 Minuten durchgeführt wird; und
einen zweiten REM-Schlafbetriebsschritt, der nach Beendigung des ersten REM-Schlafbetriebs mindestens ein Mal in Abständen von 60-80 Minuten durchgeführt wird.

14. Verfahren nach einem der Ansprüche 10 bis 13, **dadurch gekennzeichnet, dass** der REM-Schlafbetriebsschritt für 20-40 Minuten durchgeführt wird.

## Revendications

1. Climatiseur (100) comprenant une unité de climatisation (110) pour climatiser un espace intérieur, une unité d'entrée (120) recevant des signaux pour actionner l'unité de climatisation (110) et une unité de commande (130) pour commander l'unité de climatisation (110) en fonction des signaux entrés dans l'unité d'entrée (120),
dans lequel l'unité d'entrée (120) reçoit un signal de sélection d'un mode de sommeil, l'unité de commande (130) commande de sorte que lorsque l'unité d'entrée (120) reçoit le signal de sélection du mode de sommeil, l'unité de climatisation (110) réalise une phase d'endormissement, une phase de sommeil et une phase de sommeil à mouvements oculaires rapides, la phase de sommeil et la phase de sommeil à mouvements oculaires rapides pouvant étant réalisées de façon répétée en alternance, et
la phase de sommeil est réalisée pendant une durée prédéfinie à une température (T0) qui est fixée en fonction du mode de sommeil, la phase d'endormissement est réalisée pendant une durée prédéfinie à une température inférieure à la température (T0) de la phase de sommeil, la phase de sommeil à mouvements oculaires rapides est réalisée pendant une durée prédéfinie à une température supérieure à la température (T0) de la phase de sommeil, la durée prédéfinie de la phase de sommeil à mouvements oculaires rapides est inférieure à la durée prédéfinie de la phase de sommeil et la température (T0) de la phase de sommeil est maintenue constante entre les phases de sommeil à mouvements oculaires rapides.

2. Climatiseur selon la revendication 1, **caractérisé en ce que** la phase de sommeil à mouvements oculaires rapides est réalisée pendant une durée correspondant à un cycle de mouvements oculaires rapides dans un processus de réalisation du mode de sommeil.

3. Climatiseur selon l'une quelconque des revendications 1 et 2, **caractérisé en ce que** la phase de sommeil à mouvements oculaires rapides est réalisée plusieurs fois à des intervalles de temps prédéfinis.

4. Climatiseur selon l'une quelconque des revendications 1 à 2, **caractérisé en ce que** la phase de sommeil à mouvements oculaires rapides est réalisée plusieurs fois à des intervalles de 60 - 110 minutes dans un processus de réalisation de la phase de sommeil.

5. Climatiseur selon l'une quelconque des revendications 1 à 4, **caractérisé en ce que** la phase de sommeil à mouvements oculaires rapides comprend :
une première phase de sommeil à mouvements oculaires rapides qui est réalisée au moins une fois à des intervalles de 90 - 100 minutes pendant un processus de réalisation du mode de sommeil ; et
une deuxième phase de sommeil à mouvements oculaires rapides qui est réalisée au moins une fois à des intervalles de 60 - 80 minutes après la fin de la première phase de sommeil à mouvements oculaires rapides.

6. Climatiseur selon l'une quelconque des revendications 1 à 5, **caractérisé en ce que** dans la phase de sommeil à mouvements oculaires rapides, l'unité de climatisation (110) climatise l'espace intérieur à une température supérieure de 1°C à celle utilisée dans la phase de sommeil.

7. Climatiseur selon l'une quelconque des revendications 1 à 6, **caractérisé en ce que** la phase de sommeil à mouvements oculaires rapides est réalisée pendant 20 - 40 minutes.

8. Climatiseur selon l'une quelconque des revendications 1 à 7, **caractérisé en ce que** dans la phase d'endormissement, l'unité de climatisation (110) climatise l'espace intérieur à une température inférieure de 2°C à celle de la phase de sommeil.

9. Climatiseur selon l'une quelconque des revendications 1 à 7, **caractérisé en ce que** la phase d'endormissement est réalisée pendant 5 -15 minutes.

10. Procédé de commande d'un climatiseur (100) comprenant une unité de climatisation (110) pour climatiser un espace intérieur, une unité d'entrée (120) recevant des signaux pour régler la température et la durée de climatisation pour l'espace intérieur et une unité de commande (130) pour commander le fonctionnement de l'unité de climatisation (110), le procédé comprenant les étapes consistant à :
permettre à l'unité de climatisation (110) de climatiser l'espace intérieur à une température inférieure à la température de climatisation (T0) dans une étape d'endormissement ;
permettre à l'unité de climatisation (110) de climatiser l'espace intérieur à la température de climatisation (T0) dans une étape de phase de sommeil ; et
permettre à l'unité de climatisation (110) de climatiser l'espace intérieur à une température de sommeil à mouvements oculaires rapides supérieure à la température de climatisation (T0) dans une étape de phase de sommeil à mouvements oculaires rapides,
dans lequel l'étape de phase de sommeil et l'étape de phase de sommeil à mouvements oculaires rapides sont réalisées de façon répétée en alternance jusqu'à ce que la durée de la phase soit écoulée,
l'étape de phase de sommeil est réalisée pendant une durée prédéfinie à une température (T0), l'étape de phase de sommeil à mouvements oculaires rapides est réalisée pendant une durée prédéfinie, la durée prédéfinie de l'étape de phase de sommeil à mouvements oculaires rapides est inférieure à la durée prédéfinie de l'étape de phase de sommeil et la température (T0) est maintenue constante pendant l'étape de phase de sommeil entre les étapes de phase de sommeil à mouvements oculaires rapides.

11. Procédé selon la revendication 10, **caractérisé en ce que** la température d'endormissement est inférieure de 2° à la température de climatisation ; et
la température de sommeil à mouvements oculaires rapides est supérieure de 1°C à la température de climatisation.

12. Procédé selon l'une quelconque des revendications 10 et 11, **caractérisé en ce que** l'étape de phase d'endormissement est réalisée pendant 5 -15 minutes.

13. Procédé selon l'une quelconque des revendications 10 à 12, **caractérisé en ce que** l'étape de phase de sommeil à mouvements oculaires rapides comprend :
une première étape de phase de sommeil à mouvements oculaires rapides qui est réalisée au moins une fois à des intervalles de 90 - 100 minutes pendant un processus de réalisation du mode de sommeil ; et
une deuxième étape de phase de sommeil à mouvements oculaires rapides qui est réalisée au moins une fois à des intervalles de 60 - 80 minutes après la fin de la première phase de sommeil à mouvements oculaires rapides.

14. Procédé selon l'une quelconque des revendications 10 à 13, **caractérisé en ce que** l'étape de phase de sommeil à mouvements oculaires rapides est réalisée pendant 20 - 40 minutes.
